# EUROPÄISCHE PATENTSCHRIFT

(11) **EP 2 338 863 B1**
(45) Veröffentlichungstag und Bekanntmachung des Hinweises auf die Patenterteilung: **08.06.2016**
(21) Anmeldenummer: 10015601.7
(22) Anmeldetag: 14.12.2010
(51) Int. Cl.: C06B 43/00, C06C 7/00, C07D 257/06

(54) **Verfahren zur Herstellung von Bis-Tetrazolyltriazenat und Sprengstoff oder Brennstoff enthaltend bis-Tetrazolyltriazenat**
Method for producing Bis-tetrazolyltriazenate and explosive or fuel containing bis-tetrazolyltriazenate
Procédé de fabrication de Bis-tétrazolyltriazenate et explosif ou carburant contenant du bis-tetrazolyltriazenate

(30) Priorität: 23.12.2009 DE 102009060242; 23.06.2010 DE 102010024728
(43) Veröffentlichungstag der Anmeldung: 29.06.2011
(73) Patentinhaber: Diehl BGT Defence GmbH & Co. KG, 88662 Überlingen (DE)
(72) Erfinder: Hahma, Arno, 91239 Henfenfeld (DE); Karvinen, Eero, 40340 Jyväskylä (FI)
(74) Vertreter: Diehl Patentabteilung

(56) Entgegenhaltungen:
- EP-A1- 0 951 923
- WO-A2-98/22208
- GB-A- 185 555
- US-A- 1 511 771
- US-A- 1 580 572
- US-A- 2 064 817
- EULGEM P J ET AL: "New rare earth metal complexes with nitrogen-rich ligands: 5,5'-bitetrazolate and 1,3-bis(tetrazol-5-yl)triazenate - On the borderline between coordination and the formation of salt-like compounds", CHEMISTRY - A EUROPEAN JOURNAL 20080418 WILEY-VCH VERLAG DE, Bd. 14, Nr. 12, 18. April 2008 (2008-04-18), Seiten 3727-3736, XP002632488, DOI: DOI:10.1002/CHEM.200702006
- KLAPOTKE T M ET AL: "Investigations of bis(methyltetrazolyl)triazenes as nitrogen-rich ingredients in solid rocket propellants - Synthesis, characterization and properties", POLYHEDRON, PERGAMON PRESS, OXFORD, GB, Bd. 28, Nr. 1, 14. Januar 2009 (2009-01-14), Seiten 13-26, XP025840187, ISSN: 0277-5387, DOI: DOI:10.1016/J.POLY.2008.09.015 [gefunden am 2008-11-01]
- LYAKHOV A S ET AL: "Bis[1,3-bis(2-methyltetrazol-5-yl-.kappa. N4) triazenido-.kappa.N2]nickel(II)", ACTA CRYSTALLOGRAPHICA SECTION C. CRYSTAL STRUCTURE COMMUNICATIONS, MUNKSGAARD, COPENHAGEN, DK, Bd. 60, Nr. 9, 1. September 2004 (2004-09-01), Seiten M421-M422, XP008135262, ISSN: 0108-2701, DOI: DOI:10.1107/S0108270104016087 [gefunden am 2004-09-06]

## Beschreibung

Die Erfindung betrifft einen Sprengstoff oder Brennstoff enthaltend ein Gemisch aus mindestens zwei verschiedenen bis-Tetrazolyltriazenaten, eine verwendung des Sprengstoffs und des Brennstoffs sowie ein Verfahren zur Herstellung eines bis-Tetrazolyltriazenats.

Aus J. Thiele Justus Liebigs Ann. Chem. 1892, Band 270, Seiten 54 bis 63 ist die Herstellung eines Bleisalzes aus bis-Tetrazolyltriazen bekannt. Aus US 2,090,745 ist ein dieses Bleisalz enthaltender Sprengstoff bekannt.

Aus Eulgem, P. J. et al., Chem. Eur. J. 2008, Band 14, Seiten 3727 bis 3736 sind Salze von Seltenerdmetallen aus bis-Tetrazolyltriazen bekannt. Aus US 2,004,719 ist ein Kupferammoniumsalz von bis-Tetrazolyltriazen als Initialsprengstoff bekannt.

Aufgabe der vorliegenden Erfindung ist es, einen Sprengstoff oder Brennstoff sowie eine Verwendung des Sprengstoffs und des Brennstoffs und ein Verfahren zur Herstellung eines Bestandteils des Sprengstoffs und des Brennstoffs bereitzustellen.

Erfindungsgemäß wird die Aufgabe durch die Merkmale der Ansprüche 1, 7, 9 und 10 gelöst. Zweckmäßige Ausgestaltungen ergeben sich aus den Merkmalen der Ansprüche 2 bis 6, 8, 11 und 12.

Ein bis-Tetrazolyltriazenat ist zur Verwendung vorgesehen, wobei das bis-Tetrazolyltriazenat ein Guanidinsalz, ein Guanylhamstoffsalz, ein Melaminsalz oder ein Salz eines Alkalimetalls oder eines Erdalkalimetalls ist. Diese Salze stellen hochenergetische Sprengstoffe oder Brennstoffe dar, welche die ungewöhnliche Eigenschaft einer hohen thermischen Stabilität und Unempfindlichkeit gegenüber äußeren Einflüssen, insbesondere gegenüber Schlag, aufweisen.

Eine hohe thermische Stabilität im Sinne der Erfindung liegt vor, wenn eine Zersetzung erst bei Temperaturen über 280°C erfolgt. Die thermische Stabilität einzelner Salze übersteigt sogar 400°C. Die Salze sind damit als Sekundärsprengstoff oder als Zusatzmaterial in einem Initialsprengstoff zur Erhöhung der Hitzebeständigkeit bzw. Reduzierung der Empfindlichkeit geeignet. Dadurch lassen sich auch in heißer Umgebung sicher zu handhabende Sprengstoffe bereitstellen.

Weiterhin sind die Salze dazu geeignet, den Sprengstoffanteil eines Sprengstoffgemischs zu erhöhen, ohne dass das Gemisch dadurch so empfindlich wird, dass es nicht mehr sicher gehandhabt werden kann. Im Hinblick auf das Mischen mit anderen Sprengstoffen weist das bis-Tetrazolyltriazenat den Vorteil auf, dass es neutral, d. h. weder basisch noch sauer, und äußerst schwer löslich ist. Dadurch ist es sowohl mit sauren als auch mit basischen anderen Sprengstoffen gut verträglich. Es bewirkt bei einer Mischung mit anderen Sprengstoffen auch bei längerer Lagerung des resultierenden Gemischs keine Zersetzungs- und Abbaureaktionen der anderen Sprengstoffe. Darüber hinaus hat sich gezeigt, dass das erfindungsgemäße bis-Tetrazolyltriazenat im Vergleich zu herkömmlichen Sprengstoffen, wie beispielsweise Tetrazen, selbst eine extrem hohe Stabilität und Lagerfähigkeit aufweist.

Vorzugsweise handelt es sich bei dem Alkalimetall um Natrium oder Kalium und bei dem Erdalkalimetall um Magnesium, Calcium, Strontium oder Barium. Die Salze dieser Metalle weisen eine besonders hohe thermische Stabilität auf.

Die Erfindung betrifft einen Sprengstoff oder Brennstoff enthaltend ein Gemisch aus mindestens zwei verschiedenen bis-Tetrazolyltriazenaten Durch das Vorsehen eines Gemischs aus mindestens zwei verschiedenen bis-Tetrazolyltriazenaten kann ein hochenergetischer Sprengstoff oder Brennstoff bereitgestellt werden, bei dem durch das Mischungsverhältnis der bis-Tetrazolyltriazenate eine stufenlose Einstellung der Hitzebeständigkeit und Empfindlichkeit sowie der für eine Zündung des Sprengstoffs oder Brennstoffs erforderlichen Zündleistung bzw. Anzündleistung möglich ist. Dadurch ist es auch möglich, einen Initialsprengstoff ohne das darin bisher übliche Bleiazid bereitzustellen. Da die Eigenschaften des bisher üblichen Initialsprengstoffs durch den erfindungsgemäßen Sprengstoff nachgebildet werden können, sind strukturelle oder andere Änderungen in einer bisher üblichen den Sprengstoff oder Brennstoff enthaltenden Konstruktion nicht erforderlich. Der erfindungsgemäße Sprengstoff leistet durch das Vermeiden des Einsatzes von Blei einen Beitrag zum Umweltschutz.

Die Eigenschaften des Sprengstoffs, insbesondere dessen Empfindlichkeit, können/kann durch die Wahl der Kationen der in dem Gemisch enthaltenen bis-Tetrazolyltriazenate eingestellt werden. Zur Bereitstellung einer für einen Initialsprengstoff erforderlichen Empfindlichkeit ist kein Zusatz von hochkorrosivem Chlorat erforderlich. Durch die Mischung von zwei oder mehreren bis-Tetrazolyltriazenaten entstehen keine sicherheitstechnischen Probleme, weil in dem resultierenden Gemisch lediglich lonentauschreaktionen zwischen verschiedenen bis-Tetrazolyltriazenaten erfolgen. Bei sämtlichen lonenaustauschreaktionen zwischen den bis-Tetrazolyltriazenaten des Gemischs bleibt die Gesamtzusammensetzung des Gemischs auch bei langer Lagerung konstant. Bei Salzgemischen, die kein gemeinsames identisches Ion aufweisen, wie dies üblicherweise, beispielsweise bei Beimischung von Kaliumchlorat, der Fall ist, erfolgt dagegen mit längerer Lagerung durch lonenaustauschreaktionen eine Veränderung des Gemischs und damit auch der Eigenschaften.

Durch den erfindungsgemäßen Sprengstoff oder Brennstoff kann wegen dessen großer Stabilität und dessen guter Verträglichkeit mit anderen Sprengstoffen eine lange Lagerfähigkeit erreicht werden. Insbesondere wenn der erfindungsgemäße Sprengstoff als Initialsprengstoff eingesetzt wird, stellt dessen gute Verträglichkeit mit Sekundärsprengstoffen einen großen Fortschritt dar. Das zurzeit häufig als Initialsprengstoff eingesetzte Tetrazen ist dagegen sehr instabil. Das häufig als Ersatz für Bleitrizinat in Initialsprengsätzen eingesetzte Kaliumdinitrobenzofuroxan bewirkt eine starke Korrosion an Bauteilen und eine Zersetzung anderer Sprengstoffe. Eine längere Lagerung ist bei Kontakt mit den meisten Sekundärsprengstoffen daher nicht möglich.

Darüber hinaus ist herkömmlichen Zünd- und Anzündsätzen häufig Kaliumchlorat beigemischt. Kaliumchlorat ist jedoch mit allen herkömmlichen Initialsprengstoffen auf Dauer unverträglich und verursacht starke Korrosion an den Bauteilen von Sprengstoff oder Brennstoff enthaltenden Konstruktionen. Der Einsatz all dieser Stoffe kann bei Vorsehen eines erfindungsgemäßen Sprengstoffs vermieden werden. Der erfindungsgemäße Sprengstoff oder Brennstoff ist neutral und sehr schwerlöslich. Dadurch ist er sowohl mit sauren als auch mit basischen Verbindungen, insbesondere anderen Sprengstoffen, verträglich und mit diesen mischbar, ohne dass es zu Zersetzungsreaktionen kommt. Weiterhin enthalten bis-Tetrazolyltriazenate keine Halogene und verursachen keine Korrosion.

Durch den erfindungsgemäßen Sprengstoff oder Brennstoff kann der Einsatz von Blei vermieden, die Lagerfähigkeit von Sprengsätzen bei gleichzeitig hoher Zündleistung erhöht und die durch herkömmlichen Sprengstoff oder Brennstoff üblicherweise verursachte Korrosion an Bauteilen verhindert werden. Darüber hinaus ist die Herstellung von bis-Tetrazolyltriazenaten sehr einfach. Sie können aus wässrigen Lösungen ausgefällt werden. Die Herstellung von dazu erforderlichen Ausgangsstoffen ist ebenfalls aus wässriger Lösung und aus günstigen handelsüblichen Chemikalien in einer Stufe möglich. Bis-Tetrazolyltriazenate sind wesentlich einfacher herzustellen als z. B. das oben genannte Kaliumdinitrobenzofuroxan.

Ein weiterer Vorteil des erfindungsgemäßen Sprengstoffs besteht darin, dass dessen Zündleistung sehr hoch ist. Insbesondere ist sie höher als diejenige von Bleiazid. Der erfindungsgemäße Sprengstoff oder Brennstoff ist wesentlich energetischer als herkömmliche Initialsprengstoffe oder Brennstoffe.

Das Gemisch des erfindungsgemäßen Sprengstoffs oder Brennstoffs wird in Anspruch 1 definiert.

Bei dem Übergangsmetall kann es sich um Lanthan, Kupfer, Nickel, Zink, Cobalt, Mangan, Cadmium oder Chrom handeln.

Vorzugsweise ist in dem Sprengstoff oder Brennstoff kein Bleisalz von bis-Tetrazolyltriazen enthalten, um eine Bleibelastung der Umwelt zu vermeiden. Weiterhin ist es bevorzugt, wenn kein Eisensalz von bis-Tetrazolyltriazen enthalten ist, weil dieses im Vergleich zu anderen bis-Tetrazolyltriazenaten eine verhältnismäßig geringe Stabilität aufweist.

Bevorzugt ist bei dem erfindungsgemäßen Sprengstoff oder Brennstoff an mindestens einem der Tetrazolyl-Reste des bis-Tetrazolyltriazenats ein Wasserstoffrest durch eine Methylgruppe oder eine Aminogruppe substituiert.

Weiterhin ist die Verwendung des erfindungsgemäßen Sprengstoffs als Initialsprengstoff, als Sekundärsprengstoff oder als Zusatzstoff in einem Initialsprengstoff oder Sekundärsprengstoff zur Erhöhung der Hitzebeständigkeit des Initialsprengstoffs oder des Sekundärsprengstoffs vorgesehen.

Vorzugsweise enthält der Initialsprengstoff kein Chlorat und/oder Perchlorat. Da durch den erfindungsgemäßen Sprengstoff die Eigenschaften des Initialsprengstoffs so eingestellt werden können, dass dieser einem Chlorat und/oder Perchlorat enthaltendem Sprengstoff entspricht, ist das Vorsehen von Chlorat und/oder Perchlorat nicht erforderlich. Dadurch können die mit Chlorat und Perchlorat einhergehenden Probleme, insbesondere die durch Chlorat und Perchlorat bewirkte Korrosion, vermieden werden.

Besonders vorteilhaft ist die Verwendung des erfindungsgemäßen Brennstoffs als Brennstoff eines Scheinziels, wobei das bis-Tetrazolyltriazenat ein Guanidinsalz oder ein Salz eines Alkalimetalls oder eines Erdalkalimetalls ist. Dadurch, dass das bis-Tetrazolyltriazenat hoch energetisch ist und einen hohen Stickstoffgehalt und keinen Sauerstoff aufweist wird bei dessen Verbrennung eine bei Scheinzielen gewünschte große Flamme erzeugt.

Weiterhin ist erfindungsgemäß ein Verfahren zur Herstellung eines bis-Tetrazolyltriazenats mit folgenden Schritten vorgesehen:
a) Synthese eines Mono-Natriumsalzes oder eines sonstigen Mono-Alkalimetallsalzes des bis-Tetrazolyltriazens als Rohprodukt,
b) Lösen des Rohprodukts in alkalischer wässriger Lösung,
c) Ansäuern der Lösung auf einen pH-Wert zwischen 5 und 6,
d) Kühlen der Lösung auf 0,1°C bis 10°C, sofern sie diese Temperatur nicht bereits hat, und Ruhenlassen der Lösung bei dieser Temperatur,
e) Absondern des dabei gebildeten Niederschlags.

Bei dem Verfahren wird bei Schritt b) zunächst ein dreiwertiges bis-Tetrazolyltriazenat gebildet, welches bei Schritt c) zu einem einwertigen Salz umgesetzt wird. Das Absondern des gebildeten Niederschlags kann ein Waschen, beispielsweise mit einem Alkohol, insbesondere Ethanol, und gegebenenfalls ein Trocknen umfassen. Bei Durchführung des Verfahrens ist darauf zu achten, dass bei Schritt c) die Lösung nicht auf einen ph-Wert unterhalb von 5 angesäuert wird, weil dann auch vermehrt Verunreinigungen ausfallen. Diese Verunreinigungen verhindern einerseits eine definierte Zusammensetzung des erfindungsgemäßen Sprengstoffs oder Brennstoffs und sind andererseits nachteilig für die Eigenschaften des Sprengstoffs oder Brennstoffs, insbesondere für dessen Haltbarkeit.

Vorzugsweise wird der in Schritt e) abgesonderte Niederschlag in einer alkalischen wässrigen Lösung gelöst und das dabei gebildete dreiwertige bis-Tetrazolyltriazenat, insbesondere durch Zusatz eines Alkohols, vorzugsweise Ethanol, ausgefällt und abgesondert. Auch hier kann das Absondern ein Waschen und gegebenenfalls Trocknen des ausgefällten bis-Tetrazolyltriazenats umfassen.

In einem weiteren Verfahrensschritt kann eine wässrige Lösung des abgesonderten dreiwertigen bis-Tetrazolyltriazenats hergestellt werden, und eine Salzlösung mit Gegenionen zum bis-Tetrazolyltriazen-Anion, insbesondere im Überschuss, zu der Lösung zugesetzt werden. Dadurch wird bis-Tetrazolyltriazenat ausgefällt. Das ausgefällte bis-Tetrazolyltriazenat wird abgesondert. Das Absondern kann auch hier ein Waschen und gegebenenfalls Trocknen des ausgefällten bis-Tetrazolyltriazenats umfassen.

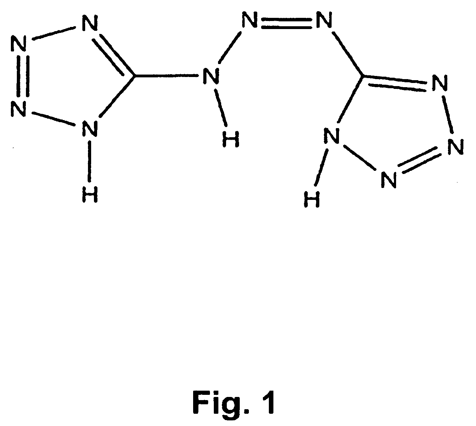

Nachfolgend wird die Erfindung anhand der Fig. 1 und von Ausführungsbeispielen erläutert.

Fig. 1 zeigt die Strukturformel von bis-Tetrazolyltriazen. Der Stoff stellt eine dreiwertige Säure dar, die mit Gegenionen schwerlösliche Salze bilden kann.

### 1. Herstellung von Mononatrium-bis-tetrazolyltriazenat

### 1.1 Synthese

144 g Aminoguanidinnitrat, 73 g Natriumacetat (die Gewichtsangaben beziehen sich jeweils auf wasserfreies Salz) und 107 g 100%ige Essigsäure werden zu ca. 300 ml Wasser zugesetzt und gerührt. Das Gemisch wird danach auf 600 ml mit Wasser aufgefüllt, wobei sich eine klare Lösung bildet. Die Lösung wird auf 5°C gekühlt. 106 g festes Natriumnitrit wird in kleinen Portionen über einer Stunde hinweg zugegeben. Die Temperatur wird während der Zugabe zwischen 5 und 13°C gehalten. Eine Stunde nach der Zugabe wird die jetzt hellorange Lösung bei 5 - 13°C mittels eines Büchnerfilter filtriert, um ausgefallene Verunreinigungen zu entfernen. Die klare hellorange Lösung wird dann über Nacht bei Raumtemperatur ohne Rühren stehen gelassen. Die Farbe der Lösung wird während der dabei stattfindenden Reaktion allmählich dunkler und es entsteht Stickstoff. Innerhalb einiger Stunden beginnt das Mononatriumsalz in Form von hellgelben Kristallen auszufallen. Am nächsten Tag ist es vollständig ausgefallen. Die Kristalle werden filtriert und zweimal mit 20 ml eiskaltem Wasser und danach mit 400 ml technischem Ethanol gewaschen. Die Ausbeute an dem dabei gewonnenen Rohprodukt beträgt 59 g (59 % der Theorie).

Statt Aminoguanidinnitrat kann auch Aminoguanidinhydrochlorid oder Aminoguanidinsulfat im gleichen Molverhältnis zum Natriumnitrit eingesetzt werden. Dadurch kann eine um ca. 10 % höhere Ausbeute erreicht werden.

### 1.2 Reinigung

Das Rohprodukt hat sich für weitere Reaktionen als zu unrein erwiesen. Es wird daher durch Umkristallisation wie folgt gereinigt:
Das trockene Rohprodukt wird in 150 ml Wasser aufgeschlämmt. Dazu wird eine Menge an Natriumhydroxid zugegeben, die theoretisch erforderlich ist, um ein Trinatriumsalz zu bilden. Das Hydroxid kann entweder als Feststoff oder als eine konzentrierte Lösung zugegeben werden. Dabei löst sich das Rohprodukt auf. Sollte es sich nicht vollständig lösen, wird weiteres Natriumhydroxid zugesetzt bis eine vollständige Lösung eingetreten ist. Nach der Zugabe wird die Lösung auf 500 ml mit destilliertem Wasser verdünnt. Die Lösung enthält das Trinatriumsalz des bis-Tetrazolyltriazens. Zu der Lösung wird über einer Stunde hinweg so viel 50%ige Essigsäure zugegeben, dass ein pH-Wert von 5 bis 5,5 erreicht wird. Dabei ist zu beachten, dass das pH nicht unter 5 fallen sollte, weil sonst ein unreines Produkt gebildet wird. Anschließend wird die Lösung in einem Eisbad gekühlt und 4 Stunden lang langsamem gerührt oder stehen gelassen. Dabei kristallisiert das Mononatrium-bis-tetrazolyltriazenat aus. Der pastellgelbe Niederschlag wird über eine Filternutsche filtriert, dreimal mit 30 ml eiskaltem Wasser gewaschen, anschließend möglichst trocken gesaugt und zum Schluss mit Ethanol gewaschen bis kein Essiggeruch des Niederschlags mehr festzustellen ist. Die Ausbeute beträgt 51 g (86 % der Theorie).

### 2. Verfahren zur Herstellung von Nickel(II)bis-tetrazolyltriazenat

10,0 g (0,042 mol) des umkristallisierten Mononatrium-bis-tetrazolyltriazenats werden in 40 ml destilliertem Wasser in einem 250 ml Becherglas eingeschlämmt. 3,5 g (0,0875 mol) Natriumhydroxid werden in 20 ml destilliertem Wasser in einem 100 ml Becher aufgelöst. Die Natriumhydroxidlösung wird in die Mononatrium-bis-tetrazolyltriazenat-Suspension eingegossen und das dunkelorange Gemisch so lange gerührt, bis sich das Mononatrium-bis-tetrazolyltriazenat vollständig gelöst hat. Diese Lösung enthält nun reines Trinatriumsalz des bis-Tetrazolyltriazenats in einem kleinen Natriumhydroxidüberschuss.

600 ml Ethanol werden in ein 1 Liter Becherglas gefüllt. Die Trinatriumsalzlösung wird über 10 Minuten hinweg bei Raumtemperatur in das Ethanol eingetropft. Der dabei gebildete hellbraune Niederschlag (reines Trinatriumsalz) wird mittels eines Büchnertrichters abfiltriert und mit 100 ml Ethanol in mehreren Portionen gewaschen, um Natriumhydroxidreste zu entfernen. Der Niederschlag wird auf ein Uhrglas aufgetragen und bei 40°C unter Vakuum getrocknet. Die Ausbeute beträgt 10,8 g (99 % der Theorie). Das Trinatriumsalz ist schwach hygroskopisch. Es soll daher in gut verschlossenen Gebinden oder als Lösung aufbewahrt werden.

10,0 g (0,04 mol) des Trinatrium-bis-tetrazolyltriazenats werden in eine 100 ml Messflasche eingewogen und auf 100 ml mit destilliertem Wasser aufgefüllt. 10 ml dieser Lösung werden in einem 250 ml Becherglas mit destilliertem Wasser auf etwa 100 ml verdünnt. 10 ml 1 molare Nickelsulfatlösung werden über 5 Minuten hinweg in diese Lösung eingetropft. Dabei bildet sich ein graugrüner Niederschlag, der durch Filtration abgesondert wird. Der Filterkuchen wird mit destilliertem Wasser und Ethanol gewaschen und bei 40°C unter Vakuum auf einem Uhrglas getrocknet. Die dabei erreichte Ausbeute ist bezogen auf das Triazenat quantitativ. Die Metallionen werden bei dem Verfahren im Überschuss eingesetzt, um das Triazenat vollständig zu fällen.

### 3. Verfahren zur Herstellung von Kupfer(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Kupfersulfatlösung verwendet. Das Produkt fällt als olivgrüner Niederschlag aus.

### 4. Verfahren zur Herstellung von Kobalt(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Kobalt(II)chloridlösung verwendet. Das Produkt fällt als rotbrauner Niederschlag aus.

### 5. Verfahren zur Herstellung von Chrom(III)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Chrom(III)chloridlösung verwendet. Das Produkt fällt als graugrüner Niederschlag aus.

### 6. Verfahren zur Herstellung von Silber-bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Silbernitratlösung verwendet. Das Produkt fällt als gelber Niederschlag aus.

### 7. Verfahren zur Herstellung von Blei(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Bleinitratlösung verwendet. Das Produkt fällt als gelber Niederschlag aus.

### 8. Verfahren zur Herstellung von Zink(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Zinkchloridlösung verwendet. Das Produkt fällt als gelber Niederschlag aus.

### 9. Verfahren zur Herstellung von Eisen(III)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Eisen(III)chloridlösung verwendet. Das Produkt fällt als schwarzer Niederschlag aus.

### 10. Verfahren zur Herstellung von Barium(II)bis-tetrazolyltriazenat

Es wird wie bei der Herstellung von Nickel(II)bis-tetrazolyltriazenat vorgegangen. Statt 1 molarer Nickelsulfatlösung werden jedoch 20 ml 0,5 molare Bariumnitratlösung verwendet. Das Produkt fällt als gelber Niederschlag aus.

### 11. Verfahren zur Herstellung von Mangan(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Mangan(II)chloridlösung verwendet. Das Produkt fällt als rosafarbener Niederschlag aus.

### 12. Verfahren zur Herstellung von Magnesium(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Magnesiumsulfatlösung verwendet. Das Produkt fällt als gelber Niederschlag aus.

### 13. Verfahren zur Herstellung von Calcium(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Calciumchloridlösung verwendet. Das Produkt fällt als blassgelber Niederschlag aus.

### 14. Verfahren zur Herstellung von Strontium(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Strontiumnitratlösung verwendet. Das Produkt fällt als blassgelber Niederschlag aus.

### 15. Verfahren zur Herstellung von Cadmium(II)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Cadmiumchloridlösung verwendet. Das Produkt fällt als gelber Niederschlag aus.

### 16. Verfahren zur Herstellung von Indium(III)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Indiumchloridlösung verwendet. Das Produkt fällt als gelber Niederschlag aus.

### 17. Verfahren zur Herstellung von Lanthan(III)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Lanthannitratlösung verwendet. Das Produkt fällt als gelber Niederschlag aus.

### 18. Verfahren zur Herstellung von Bismut(III)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Vismutnitratlösung verwendet. Das Produkt fällt als gelber Niederschlag aus.

### 19. Verfahren zur Herstellung von Aluminium(III)bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Aluminiumchloridlösung verwendet. Das Produkt zersetzt in der Lösung ohne Niederschlag. Die Lösung wird durch das Aluminiumchlorid zu sauer.

### 20. Verfahren zur Herstellung von Guanidin(bis-tetrazolyltriazenat)

Es wird wie bei der Herstellung von Nickel(II)bis-tetrazolyltriazenat vorgegangen. Statt 1 molarer Nickelsulfatlösung werden jedoch 10 ml 1 molare Guanidinnitratlösung verwendet. Das Produkt fällt als gelber Niederschlag aus.

### 21. Verfahren zur Herstellung von Aminoguanidin-bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Aminoguanidinnitratlösung verwendet. Das Produkt fällt als gelber Niederschlag aus.

### 22. Verfahren zur Herstellung von Diaminoguanidin-bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Diaminoguanidinnitratlösung verwendet. Das Produkt fällt als gelber Niederschlag aus.

### 23. Verfahren zur Herstellung von Triaminoguanidin-bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Triaminoguanidinhydrochloridlösung verwendet. Das Produkt fällt als gelber Niederschlag aus.

### 24. Verfahren zur Herstellung von Melaminium-bis-tetrazolyltriazenat

Es wird wie bei der Herstellung von Nickel(II)bis-tetrazolyltriazenat vorgegangen. Statt 1 molarer Nickelsulfatlösung werden jedoch 100 ml 0,1 molare Melaminhydrochloridlösung verwendet. Das Produkt fällt als blassgelber Niederschlag aus.

### 25. Verfahren zur Herstellung von Hydrazinium-bis-tetrazolyltriazenat

Es wird wie bei der Herstellung von Nickel(II)bis-tetrazolyltriazenat vorgegangen. Statt 1 molarer Nickelsulfatlösung werden jedoch 10 ml 1 molare Hydrazinsulfatlösung verwendet. Das Produkt fällt als hellgelber Niederschlag aus.

### 26. Verfahren zur Herstellung von Guanylharnstoff-bis-tetrazolyltriazenat

Es wird wie bei der Herstellung von Nickel(II)bis-tetrazolyltriazenat vorgegangen. Statt 1 molarer Nickelsulfatlösung werden jedoch 100 ml 0,1 molare Guanylharnstoffhydrochloridlösung verwendet. Das Produkt fällt als gelber Niederschlag aus.

### 27. Verfahren zur Herstellung von Ammonium-bis-tetrazolyltriazenat

Es wird wie bei der Herstellung von Nickel(II)bis-tetrazolyltriazenat vorgegangen. Statt 1 molarer Nickelsulfatlösung werden jedoch 100 ml 0,1 molare Ammoniumsulfatlösung verwendet. Das Produkt fällt als gelber Niederschlag aus.

### 28. Sprengstoff mit einem Gemisch aus zwei verschiedenen bis-Tetrazolyltriazenaten

### 28.1 Unempfindliches Gemisch

Es wurde ein Gemisch hergestellt aus 37 Gewichtsprozent (Gew.-%) Strontiumnitrat, 13 Gew.-% Aluminiumpulver, 10 Gew.-% amorphem Bor mit einer Korngröße von unter 5µm, 30 Gew.-% Barium(III)bis-tetrazolyltriazenat und 10 Gew.-% Kupfer(II)bis-tetrazolyltriazenat. Das Gemisch hat sich als verhältnismäßig unempfindlich erwiesen. Es weist eine Reibempfindlichkeit von etwa 20 N auf.

### 28.2 Empfindliche Gemische

Es wurde ein Gemisch hergestellt aus 37 Gew.-% Strontiumnitrat, 13 Gew.-% Aluminiumpulver, 10 Gew.-% amorphem Bor mit einer Korngröße von unter 5µm, 15 Gew.-% Barium(III)bis-tetrazolyltriazenat und 25 Gew.-% Kupfer(II)bis-tetrazolyltriazenat. Das Gemisch hat eine für viele Anwendungen geeignete Empfindlichkeit. Es weist eine Reibempfindlichkeit von etwa 2,5 N auf.

Weiterhin wurde ein Gemisch hergestellt aus 37 Gew.-% Strontiumnitrat, 13 Gew.-% Aluminiumpulver, 10 Gew.-% amorphem Bor mit einer Korngröße von unter 5µm, 5 Gew.-% Barium(III)bis-tetrazolyltriazenat und 35 Gew.-% Kupfer(II)bis-tetrazolyltriazenat. Das Gemisch hat sich als verhältnismäßig empfindlich erwiesen. Es weist eine Reibempfindlichkeit von etwa 0,4 N auf.

Darüber hinaus wurde ein Gemisch hergestellt aus 37 Gew.-% Strontiumnitrat, 13 Gew.-% Aluminiumpulver, 10 Gew.-% amorphem Bor mit einer Korngröße von unter 5µm, 10 Gew.-% Barium(III)bis-tetrazolyltriazenat und 30 Gew.-% Kupfer(II)bis-tetrazolyltriazenat. Das Gemisch hat sich ebenfalls als verhältnismäßig empfindlich erwiesen. Es weist eine Reibempfindlichkeit von etwas 1 N auf.

Bei allen oben aufgeführten Beispielen eines Sprengstoffs mit einem Gemisch aus zwei verschiedenen bis-Tetrazolyltriazenaten wird ein gleiches Gemisch aus Aluminium, Bor und Strontiumnitrat eingesetzt. Der Gesamtgehalt an Barium(III)bis-tetrazolyltriazenat und Kupfer(II)bis-tetrazolyltriazenat beträgt stets 40%, wobei das Mischungsverhältnis der beiden bis-Tetrazolyltriazenate variiert. Durch die Wahl des Mischungsverhältnisses lässt sich die Empfindlichkeit des Sprengstoffs oder Brennstoffs einstellen. Die Empfindlichkeit ist umso höher, je höher der Gehalt an Kupfer(II)bis-tetrazolyltriazenat ist.

## Patentansprüche

1. Sprengstoff oder Brennstoff enthaltend
ein Gemisch aus mindestens zwei verschiedenen bis-Tetrazolyltriazenaten, wobei das Gemisch
mindestens ein bis-Tetrazolyltriazenat, wobei das bis-Tetrazolyltriazenat ein Guanidinsalz, ein Guanylharnstoffsalz, ein Melaminsalz, ein Salz eines Alkalimetalls oder eines Erdalkalimetalls oder ein Na-, K-, Mg-, Ca-, Sr- oder Ba-Salz ist,
und/oder
mindestens ein bis-Tetrazolyltriazenat eines Übergangsmetalls, ein Mono-, Di- oder Triaminoguanidinsalz, ein Ammoniaksalz oder ein Hydrazinsalz
und/oder
mindestens ein bis-Tetrazolyltriazenat eines Metalls enthält.

2. Sprengstoff oder Brennstoff nach Anspruch 1,
wobei das Übergangsmetall ein inneres Übergangsmetall ist.

3. Sprengstoff oder Brennstoff nach einem der vorhergehenden Ansprüche,
wobei das Übergangsmetall La, Cu, Ni, Zn, Co, Mn, Cd oder Cr ist.

4. Sprengstoff oder Brennstoff nach einem der vorhergehenden Ansprüche,
wobei das Metall In oder Al ist.

5. Sprengstoff oder Brennstoff nach einem der vorhergehenden Ansprüche,
wobei kein Bleisalz und/oder Eisensalz von bis-Tetrazolyltriazen enthalten ist.

6. Sprengstoff oder Brennstoff nach einem der vorhergehenden Ansprüche,
wobei an mindestens einem der Tetrazolyl-Reste des bis-Tetrazolyltriazenats ein Wasserstoffrest durch eine Methylgruppe oder eine Aminogruppe substituiert ist.

7. Verwendung des Sprengstoffs nach einem der Ansprüche 1 bis 6 als Initialsprengstoff, als Sekundärsprengstoff oder als Zusatzstoff in einem Initialsprengstoff oder Sekundärsprengstoff zur Erhöhung von dessen Hitzebeständigkeit.

8. Verwendung nach Anspruch 7,
wobei der Initialsprengstoff kein Chlorat und/oder Perchlorat enthält.

9. Verwendung des Brennstoffs nach Anspruch 1 als Brennstoff eines Scheinziels,
wobei das bis-Tetrazolyltriazenat ein Guanidinsalz oder ein Salz eines Alkalimetalls oder eines Erdalkalimetalls ist.

10. Verfahren zur Herstellung eines bis-Tetrazolyltriazenats mit folgenden Schritten:
a) Synthese eines Mono-Natriumsalzes oder eines sonstigen Mono-Alkalimetallsalzes des bis-Tetrazolyltriazens als Rohprodukt,
b) Lösen des Rohprodukts in alkalischer wässriger Lösung,
c) Ansäuern der Lösung auf einen pH-Wert zwischen 5 und 6,
d) Kühlen der Lösung auf 0,1°C bis 10°C, sofern sie diese Temperatur nicht bereits hat, und Ruhenlassen der Lösung bei dieser Temperatur,
e) Absondern des dabei gebildeten Niederschlags.

11. Verfahren nach Anspruch 10,
wobei der in Schritt e) abgesonderte Niederschlag in einer alkalischen wässrigen Lösung gelöst wird und das dabei gebildete dreiwertige bis-Tetrazolyltriazenat, insbesondere durch Zusatz eines Alkohols, vorzugsweise Ethanol, ausgefällt und abgesondert wird.

12. Verfahren nach Anspruch 11,
wobei eine wässrige Lösung des abgesonderten dreiwertigen bis-Tetrazolyltriazenats hergestellt wird, eine Salzlösung mit Gegenionen zum bis-Tetrazolyltriazen-Anion, insbesondere im Überschuss, zu der Lösung zugesetzt
wird, um bis-Tetrazolyltriazenat auszufällen und das ausgefällte bis-Tetrazolyltriazenat abgesondert wird.

## Claims

1. Explosive or fuel containing
a mixture of at least two different bistetrazolyltriazenates, wherein the mixture contains
at least one bistetrazolyltriazenate, which is a guanidine salt, a guanylurea salt, a melamine salt, a salt of an alkali metal or an alkaline earth metal or an Na, K, Mg, Ca, Sr or Ba salt,
and/or
at least one bistetrazolyltriazenate of a transition metal, a monoaminoguanidine, diaminoguanidine or triaminoguanidine salt, an ammonium salt or a hydrazine salt
and/or
at least one bistetrazolyltriazenate of a metal.

2. Explosive or fuel according to Claim 1, wherein the transition metal is an inner transition metal.

3. Explosive or fuel according to either of the preceding claims, wherein the transition metal is La, Cu, Ni, Zn, Co, Mn, Cd or Cr.

4. Explosive or fuel according to any of the preceding claims, wherein the metal is In or Al.

5. Explosive or fuel according to any of the preceding claims, wherein no lead salt and/or iron salt of bistetrazolyltriazene is present.

6. Explosive or fuel according to any of the preceding claims, wherein a hydrogen radical on at least one of the tetrazolyl radicals of bistetrazolyltriazenate is replaced by a methyl group or an amino group.

7. Use of the explosive according to any of Claims 1 to 6 as primary explosive, secondary explosive or as additive in a primary explosive or secondary explosive to increase the heat resistance thereof.

8. Use according to Claim 7, wherein the primary explosive does not contain any chlorate and/or perchlorate.

9. Use of the fuel according to Claim 1 as fuel of a decoy, wherein the bistetrazolyltriazenate is a guanidine salt or a salt of an alkali metal or an alkaline earth metal.

10. Process for preparing a bistetrazolyltriazenate, which comprises the following steps:
a) synthesizing a monosodium salt or another monoalkali metal salt of bistetrazolyltriazene as crude product,
b) dissolving the crude product in an alkaline aqueous solution,
c) acidifying the solution to a pH in the range from 5 to 6,
d) cooling the solution to from 0.1°C to 10°C, if it is not already at this temperature, and allowing the solution to stand at this temperature,
e) isolating the precipitate formed.

11. Process according to Claim 10, wherein the precipitate isolated in step e) is dissolved in an alkaline aqueous solution and the trivalent bis-tetrazolyltriazenate formed is precipitated, in particular by addition of an alcohol, preferably ethanol, and isolated.

12. Process according to Claim 11, wherein an aqueous solution of the isolated trivalent bistetrazolyltriazenate is prepared, a salt solution containing counterions to the bistetrazolyltriazene anion is added to the solution, in particular in excess, to precipitate bistetrazolyltriazenate and the precipitated bistetrazolyltriazenate is isolated.

## Revendications

1. Explosif ou combustible, contenant
un mélange d'au moins deux bis-tétrazolyltriazénates différents, le mélange contenant
au moins un bis-tétrazolyltriazénate, le bis-tétrazolyltriazénate étant un sel de guanidine, un sel de guanylurée, un sel de mélamine, un sel d'un métal alcalin ou d'un métal alcalino-terreux ou un sel de Na, K, Mg, Ca, Sr ou Ba,
et/ou
au moins un bis-tétrazolyltriazénate d'un métal de transition, un sel de mono-, di- ou triaminoguanidine, un sel d'ammoniac ou un sel d'hydrazine,
et/ou
au moins un bis-tétrazolyltriazénate d'un métal.

2. Explosif ou combustible selon la revendication 1, dans lequel le métal de transition est un métal de transition intérieur.

3. Explosif ou combustible selon l'une quelconque des revendications précédentes, dans lequel le métal de transition est La, Cu, Ni, Zn, Co, Mn, Cd ou Cr.

4. Explosif ou combustible selon l'une quelconque des revendications précédentes, dans lequel le métal est In ou Al.

5. Explosif ou combustible selon l'une quelconque des revendications précédentes, dans lequel aucun sel de plomb et/ou sel de fer de bis-tétrazolyltriazène n'est contenu.

6. Explosif ou combustible selon l'une quelconque des revendications précédentes, dans lequel un radical hydrogène est remplacé par un groupe méthyle ou un groupe amino sur au moins un des radicaux tétrazolyle du bis-tétrazolyltriazénate.

7. Utilisation de l'explosif selon l'une quelconque des revendications 1 à 6 en tant qu'explosif initial, en tant qu'explosif secondaire ou en tant qu'additif dans un explosif initial ou un explosif secondaire pour augmenter sa résistance à la chaleur.

8. Utilisation selon la revendication 7, dans laquelle l'explosif initial ne contient pas de chlorate et/ou de perchlorate.

9. Utilisation du combustible selon la revendication 1 en tant que combustible d'une cible fictive, le bis-tétrazolyltriazénate étant un sel de guanidine ou un sel d'un métal alcalin ou d'un métal alcalino-terreux.

10. Procédé de fabrication d'un bis-tétrazolyltriazénate comprenant les étapes suivantes :
a) la synthèse d'un sel de mono-sodium ou d'un autre sel de mono-métal alcalin du bis-tétrazolyltriazène en tant que produit brut,
b) la dissolution du produit brut dans une solution aqueuse alcaline,
c) l'acidification de la solution à un pH compris entre 5 et 6,
d) le refroidissement de la solution à 0,1 °C à 10 °C, si elle ne présente pas déjà cette température, et le repos de la solution à cette température,
e) la séparation du précipité formé.

11. Procédé selon la revendication 10, dans lequel le précipité séparé à l'étape e) est dissous dans une solution aqueuse alcaline et le bis-tétrazolyltriazénate trivalent formé est précipité, notamment par ajout d'un alcool, de préférence l'éthanol, et séparé.

12. Procédé selon la revendication 11, dans lequel une solution aqueuse du bis-tétrazolyltriazénate trivalent séparé est fabriquée, une solution saline contenant des contre-ions pour l'anion bis-tétrazolyltriazène, notamment en excès, est ajoutée à la solution, afin de précipiter le bis-tétrazolyltriazénate, et le bis-triazolyltriazénate précipité est séparé.
